# EUROPÄISCHE PATENTSCHRIFT

(11) **EP 1 529 494 B1**
(45) Veröffentlichungstag und Bekanntmachung des Hinweises auf die Patenterteilung: **12.01.2011**
(21) Anmeldenummer: 04024884.1
(22) Anmeldetag: 20.10.2004
(51) Int. Cl.: A61B 17/88, A61F 2/46

(54) **Instrument zur Behandlung von osteopathisch veränderten Bereichen von menschlichen oder tierischen Knochen**
Instrument for treatment of regions of human or animal bones
Instrument pour le traitement de portions osseuses humaines ou animales

(30) Priorität: 08.11.2003 DE 20317230 U
(43) Veröffentlichungstag der Anmeldung: 11.05.2005
(73) Patentinhaber: Buchholz, Jürgen, Dr., 42929 Wermelskirchen (DE)
(72) Erfinder: Buchholz, Jürgen, Dr., 42929 Wermelskirchen (DE)
(74) Vertreter: Zenz

(56) Entgegenhaltungen:
- WO-A-00/44319
- DE-A1- 10 003 331
- US-A- 5 059 193
- US-A1- 2002 026 197

## Beschreibung

Die Erfindung betrifft ein Instrument zur Behandlung von osteopathisch veränderten Bereichen der Wirbelsäule sowie von Röhrenknochen, welches ein am vorderen Ende eines schlauch- oder röhrchenartigen Führungselements angeordnetes und mittels des Führungselements durch eine minimalinvasiv in der Kortikalis des Knochens erzeugte Öffnung ins Knocheninnere in den osteopathischen Knochenbereich einführ- und dort durch Manipulation von außen in seinen äußeren Abmessungen vergrößerbares Verformungselement aufweist, welches bei Abmessungs-Vergrößerung durch Verdrängung von osteopathischem Knochenmaterial einen Hohlraum zu bilden vermag, in welchen aushärtendes oder abbindendes Knochen- und/oder Knochenersatzmaterial in noch bildsamen Zustand einbringbar ist, welches nach dem Abbinden oder Aushärten den osteopathischen Knochenbereich stabilisiert, wobei das Verformungselement eine Vielzahl von in ihrem vorderen und rückwärtigen Endbereich gegen radiale Aufweitung miteinander verbundenen Abschnitte aus draht-, folien- oder dünnem plattenförmigen Material aufweist, welche in dem nicht in der äußeren Abmessung vergrößerten Zustand des Verformungselements einen radialen Außendurchmesser aufweisen, welcher ein Einführen durch den lichten Innendurchmesser des schlauch- oder röhrchenartigen Führungselements erlaubt, und mit Mitteln zur Annäherung der miteinander verbundenen vorderen und rückwärtigen Endbereiche der Abschnitte des Verformungselements derart versehen ist, dass sich diese zwischen ihren Endbereichen im Sinne einer radialen Vergrößerung des Durchmessers des Verformungselements verformen.

Bei der Versorgung von Osteopathien, z.B. osteoporotischen Veränderungen der Wirbelsäule sowie Röhrchenknochen, aber auch bei Knochenfrakturen sind sowohl in der Human- als auch der Veterinärmedizin in neuerer Zeit Verfahren entwickelt worden, bei
denen zur Stabilisierung Knochenersatzstoffe (Hydroxylapathit, Knochenzement etc.) in die erkrankten Bereiche eingebracht werden, wobei mittels spezieller Instrumentensätze ein Zugang zum erkrankten Knochenbereich geschaffen und dann durch dort zur Einwirkung gebrachte Instrumente ein Hohlraum geschaffen, in welchen der stabilisierende Knochenersatzstoff über Kanülen oder Lumina in noch bildsamem Zustand eingebracht wird. Nach dem Erstarren oder Abbinden ist der erzeugte erkrankte Knochenbereich dann durch den verfestigten Knochenersatzwerkstoff stabilisiert. Insbesondere im Zusammenhang mit osteoporotischen Veränderungen der Wirbelsäule sind solche Verfahren einschließlich der zur Durchführung der Behandlung einzusetzenden Instrumentensätze bekannt. In der WO 00/09024 sind solche Systeme und Verfahren zur Einbringung von Material in Knochen beschrieben. Zur Erzeugung des Hohlraums im erkrankten Knochenbereich wird dabei ein am Ende einer Kanüle angeordnetes, durch Einbringen eines Druckmediums über die Kanüle volumenvergrößerbares hohlraumformendes Instrument angeordnet, welches in den erkrankten Bereich eingeführt wird. Durch Einbringen von Druckmedium über die Kanüle wird dann das im erkrankten Bereich befindliche Knochenmaterial zusammengepresst und durch das sich erweiternde Element beiseite geschoben, so dass der Hohlraum entsteht. Das Instrument wird dann insgesamt zurückgezogen und der Hohlraum kann - gegebenenfalls nach vorheriger Ausspülung des ursprünglich dort enthaltenen und zur Seite geschobenen Knochenmaterials - über eine anschließend eingeführte Kanüle mit dem in noch bildsamem Zustand befindlichen Knochenersatzmaterial gefüllt werden, wofür am äußeren Ende dieser Kanüle dann in der Regel eine mit dem Knochenersatzmaterial gefüllte Spritze oder Spritzpistole angesetzt und betätigt wird. Das den Hohlraum erzeugende aufweitbare Element wird also vor der Befüllung des erzeugten Hohlraums mit Knochenersatzmaterial wieder aus dem Knochen zurückgezogen wird.

Zur Stabilisierung von in der Wirbelsäule aufeinander folgenden Wirbeln mit degenerierten Bandscheiben und dadurch hervorgehobenen Beschwerden ist in der US-A-5059193 (Basis für des Oberbegriff des Anspruchs 1) ein expandierbares Implantat der eingangs erwähnten Art beschrieben, welches in nicht expandiertem Zustand durch eine zuvor in den zwischen den Wirbeln bestehenden Bereich eingebrachten Bohrung einführt und dann - durch Manipulation von außerhalb des Körpers - soweit aufweitbar ist, dass die einander zugewandten, in den Wirbel-Stimflächen zusätzlich mit pfannenartigen Aufnahmen versehenen Wirbel in einem vorgegebenen Abstand gehalten sind, worauf zusätzliche zu einer Schlämme mit fein verkleinerten Partikeln von Knochenrindenmaterial oder porösen Knochenschnitzeln aufbereitetes Füllmaterial von außen in das expandierte Implantat eingebracht wird, welches die Fusion zwischen den zu stabilisierenden Wirbeln erleichtert. Als Instrument zur Verdrängung von osteopathisch verändertem Knochenmaterial ist dieses Implantat nicht bestimmt.

Aus der WO 00/44319 ist für den gleichen Zweck wie das vorerwähnte Implantat ein expandierbares, als Zwischenwirbeldistanzstück bezeichnetes Implantat bekannt, bei welchem das im Einführungszustand langgestreckte hülsenförmige und in gleichmäßigen radialen und axialen Abständen mit Schlitzen versehene Distanzstück mittels einer beim Einführen in den Zwischenwirbelbereich über das Distanzstück geschobenen hülsenförmigen Verformungshilfe zu einer Vielzahl von in Umfangs- und Längsrichtung des Distanzstücks zueinander versetzten radial vortretenden spitzenverformten Hülsenbereichen erzeugt wird, indem nach jedem Verschiebungsschritt des Verformungswerkzeugs der vor der Stirnkante des Werkzeugs liegende Distanzstück-Bereich mittels eines durch das Distanzstück geführte inneren Zugteils entlang der durch die Schlitze im Distanzstück gegebenen Bereichen ausgeknickt und so den Durchmesser vergrößernde Spitzen oder Dorne gebildet werden, die sich auf den Stirnflächen der zu distanzierenden Wirbel abstützen.

Demgegenüber liegt der Erfindung die Aufgabe zugrunde, ein verbessertes Instrument zur Erzeugung von Hohlräumen in osteopathischen Knochenbereichen ganz allgemein - gegebenenfalls auch im Bereich von durch osteopatische Veränderungen verursachten Knochenbrüchen - anzugeben, welches grundsätzlich auch die Möglichkeit eröffnet, dass es nach dem Einführen in den
zu behandelnden Bereich und der Bildung des Hohlraums in diesem verbleiben kann, d.h. nicht durch die zuvor erzeugten Kanäle im Knochen und in darüber liegenden Weichteilen des Patienten zurückgezogen werden muss, wodurch dann also auch die Übertragung von Keimen aus dem erkrankten Bereich in noch nicht befallene Bereiche vermieden werden kann.

Ausgehend von einem Instrument der eingangs erwähnten Art wird diese Aufgabe erfindungsgemäß dadurch gelöst, dass das von lediglich an ihren gegenüberliegenden Endbereichen gegen radiale Aufweitung der miteinander verbundenen Abschnitten gebildete Verformungselement in seiner im Ausgangszustand langgestreckten Form in zurückgezogener Position in einem am vorderen Ende des schlauch- oder röhrchenartigen Führungselements vorgesehenen schlauch- oder rohrförmigen Vorschubelement und aus der zurückgezogen Position, in eine aus dem freien Ende des Vorschubelements vorstehende Position verschiebbar angeordnet ist, und dass das Vorschubelement seinerseits aus einer in das Ende des Führungselements zurückgezogenen in eine aus dem freien Ende des Führungselements vorgeschobene Position verschiebbar im Führungselement angeordnet ist.

In vorteilhafter Weiterbildung der Erfindung wird das Verformungselement dabei von einem strumpfartigen Netz von Metalldrähten gebildet, die im Bereich ihres vorderen und rückwärtigen Endes jeweils gegen radiale Aufweitung gesichert miteinander verbunden sind.

Alternativ kann das Verformungselement auch von einer Anzahl von in Umfangsrichtung zueinander versetzten langgestreckten Folienabschnitten gebildet werden, die an ihrem vorderen und rückwärtigen Endbereich gegen radiale Aufweitung gesichert miteinander verbunden sind. Sowohl das von einem strumpfartigen Netz von Metalldrähten als auch das von solchen langgestreckten metallischen Folienabschnitten gebildete Verformungselement sind dann lediglich in ihren gegenüberliegenden Endbereichen radial zusammengehalten, während die dazwischen liegenden Bereiche so verformbar sind, dass insgesamt eine radiale Aufweitung erfolgt.

Die Draht- oder Folienabschnitte des Verformungselements werden dabei zweckmäßig aus einem hochfesten Metall, vorzugsweise Titan oder einer Titanlegierung hergestellt. Da das Verformungselement auch nach der Einbringung des Knochenersatzmaterials im behandelten Knochenbereich verbleiben kann, bilden die hochfesten Draht- oder Folienabschnitte dann eine zusätzliche Armierung im behandelten Bereich eingebrachten Knochenersatzmaterials , wodurch die Belastbarkeit des Knochens zusätzlich erhöht wird.

Alternativ können die das Verformungselement bildenden Materialabschnitte auch aus hochgereinigtem Glas, vorzugsweise Quarzglas, hergestellt sein. Bei hinreichend geringer Bemessung der Materialstärke der Materialabschnitte weisen diese auch bei Herstellung aus Glas die für die Aufweitung des Verstellelements erforderliche Verformbarkeit auf.

Eine weitere Möglichkeit besteht darin, die Materialabschnitte des Verformungselements aus thermoverformbarem Metall, vorzugsweise einem Edelmetall, herzustellen, um die Aufweitung des Verstellelements durch Wärmezufuhr in den Materialabschnitten auszulösen. Die Materialabschnitte können dabei zweckmäßig als Bimetallelement ausgebildet sein. Eine weitere Möglichkeit besteht darin, die Materialabschnitte des Verformungselements aus einem Metall herzustellen, welches Memory-Effekt-Materialeigenschaften aufweist.

Am oder im Verformungselement kann dann eine an eine äußere Temperaturmess- und/oder -verarbeitungseinrichtung angeschlossene Temperatursonde und/oder ein an eine äußere Druckmess- und/oder -verarbeitungseinrichtung angeschlossene Drucksonde vorgesehen sein. Diese Drucksonde weist dann mit Vorteil einen piezoelektrischen Druckaufnehmer auf.

Um die für die Ausbildung des Hohlraums erforderliche radiale Aufweitung des Verformungselements zu ermöglichen, kann die Ausgestaltung mit Vorteil so getroffen sein, dass das Verformungselement von einem an dessen vorderem, gegen radiale Aufweitung gesicherten Endbereich befestigten und durch den rückwärtigen Endbereich verschieblich bis aus dem äußeren Ende des schlauch- oder röhrchenförmigen Führungselements herausgeführtes Zugorgan durchsetzt wird. Durch Ausübung eines Zugs auf das äußere Ende des Zugorgans wird dann der vordere Endbereich an den rückwärtigen Endbereich angenähert, wodurch sich die dazwischen liegenden Bereiche des Verformungselements radial aufgeweitet werden und unter Verdrängung des zu ersetzenden, beispielsweise osteoporösen und daher nicht belastbaren oder bereits zerstörten Knochengewebes den gewünschten Hohlraum bilden.

Das Zugorgan kann dann in seinem im Ausgangszustand innerhalb des Verformungselements verlaufenden Bereich mit einer Sollbruchstelle versehen sein, so dass es nach Erzeugung des Hohlraums durch Ausübung einer höhten Zugspannung im Sollbruchbereich abgeschert werden kann.

Alternativ kann das Zugorgan auch mittels einer lösbaren Kupplung am vorderen Endbereich des Verformungsorgans befestigt sein, wobei hier eine kurze Gewindeverbindung oder eine einfache Bajonettkupplung ist, so dass das Lösen des Zugorgans durch Verdrehung relativ zum Verformungselement möglich ist.

Um das Verformungselement beim Einführen in den zu behandelnden Bereich in dem langgestreckten Ausgangszustand geringen Durchmesser zu sichern, kann in erfindungsgemäßer Weiterbildung das Verformungselement in seiner im Ausgangszustand langgestreckten Form in einem am vorderen Ende des schlauch- oder röhrchenartigen Führungselements vorgesehenen schlauch- oder rohrförmigen Vorschubelements gehalten sein, welches in das Führungselement zurück- und aus dem Führungselement herausziehbar ist. Beim Einführen ist dadurch sichergestellt, dass das aus dem Führungselement vortretende Vorschubelement eine versehentliche Verformung und Aufweitung des Verformungselements verhindert. Bei Erreichen der bestimmungsgemäßen Behandlungsstellung wird das Vorschubelement dann ins schlauch- oder röhrchenartige Führungselement zurückgezogen, so dass die aufweitbaren Bereiche des Verformungselements frei werden und durch anschließendes Zurückziehen des Zugorgans aufweitbar sind.

Bei Verwendung eines solchen zurückziehbaren schlauch- oder röhrchenartigen Führungselements können die das Verstellelement bildenden Materialabschnitte auch aus einem biegeelastischen Material hergestellt sein, welches in unverformtem Zustand eine der radial aufgeweiteten Form des Verstellelements entsprechende Form haben und die beim Einführen und Vorschieben des Instruments in den erkrankten Knochenbereich unter elastischer Streckung zusammengehalten werden.

Die Erfindung ist in der folgenden Beschreibung zweier Ausführungsbeispiele in Verbindung mit der Zeichnung näher erläutert, und zwar zeigt:
- Fig. 1: einen Längsmittelschnitt durch das vordere Ende ei- nes mit einem in der erfindungsgemäßen Weise als strumpfartiges Netz von Metalldrähten ausgebildete Verformungselement im langgestreckten Aus- gangszustand als erstes Ausführungsbeispiel;
- Fig. 2: eine der Figur 1 entsprechende Schnittansicht, in welcher das Verformungselement in dem nach reichen des Behandlungsbereichs zur Erzeugung eines Hohlraums radial aufgeweiteten Zustand dar- gestellt ist;
- Fig. 3: eine Seitenansicht eines zweiten Ausführungsbei- spiels des Verformungselements im langgestreck- ten Ausgangszustand, bei welchem anstelle eines strumpfartigen Netzes von Metalldrähten eine Viel- zahl von langgestreckten Folienabschnitten aus Metall als radial aufweitbare Elemente vorgesehen sind;
- Fig. 4: eine Schnittansicht entlang der Pfeile 4-4 in Figur 3;
- Fig. 5: eine Seitenansicht des Verformungselements ge- mäß Figur 3 in dem nach Erreichen des Behand- lungsbereichs zur Erzeugung eines Hohlraums ra- dial aufgeweiteten, im Durchmesser vergrößerten Zustand;
- Fig. 6: eine Schnittansicht, gesehen in Richtung der Pfeile 6-6 in Figur 5; und
- Fig. 7: eine Seitenansicht eines der für das Verformungs- element verwendeten Folienabschnitte.

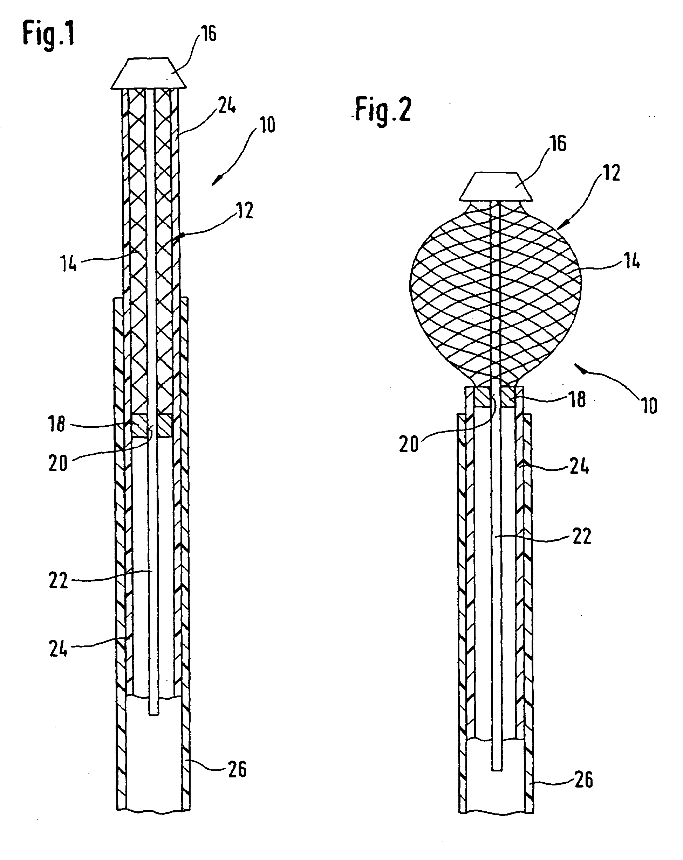

In den Figuren 1 und 2 ist ein in seiner Gesamtheit mit 10 bezeichnetes erstes Ausführungsbeispiel eines in der erfindungsgemäßen Weise ausgebildeten Instruments zur Behandlung von osteopathisch veränderten Bereichen der Wirbelsäule sowie von Röhrenknochen gezeigt, welches über ein durch eine minimal-invasiv in der Kordikalis des Knochens erzeugte Öffnung ins Knocheninnere einführbar ist.

Das Instrument 10 weist ein in dem in Figur 1 gezeigten Ausgangszustand langgestrecktes, durchmesservergrößerbares Verformungselement 12 auf, welches im dargestellten Fall von einem strumpfartigen Netz 14 aus Drähten aus einer geeigneten Metalllegierung, vorzugsweise einer Titanlegierung gebildet wird. Die Einzeldrähte dieses Netzes 14 sind in ihrem vorderen und rückwärtigen Endbereich in Endkappen 16 bzw. 18 befestigt und so in diesen Endbereichen gegen radiale Aufweitung des Netzes gesichert gehalten. Das strumpfartige Netz 14 wird von einem an der vorderen Endkappe 16 befestigten, durch das Netz 14 und eine Bohrung 20 in der rückwärtigen Endkappe 18 hindurchgeführten langgestreckten Zugorgan 22 durchsetzt, welches so lang bemessen ist, dass es sich bis zum bzw. aus dem äußeren Ende eines langgestreckten rohr- oder schlauchförmigen Vorschubelements 24 erstreckt. In dem vorderen Ende dieses rohr- oder schlauchförmigen Vorschubelements 24 ist das Instrument 10 in der Ausgangsstellung aufgenommen, wobei sich der im Durchmesser etwas vergrößerte äußere Rand der vorderen Endkappe 16 auf der Stirnkante des Vorschubelements abstützt.

Das Einführen des Instruments 10 in den zu behandelnden Bereich des Knochens erfolgt durch ein weiteres langgestrecktes rohr- oder schlauchförmiges Führungselement 26 in Form zum Beispiel einer Kanüle oder eines Lumens, dessen lichter Innendurchmesser im Wesentlichen dem Außendurchmesser des Vorschubelements 24 entspricht. Das Führungselement 26 ist zuvor von außerhalb des Körpers durch die in der Kortikalis des Knochens erzeugte Öffnung ins Innere des Knochens geführt. Vom äußeren Ende her wird dann also das Vorschubelement 24 mit dem eingesetzten Instrument durch das Führungselement 26 eingeschoben, und zwar so weit, dass die rückwärtige Endkappe 18 des noch im vorderen Ende des Vorschubelements 24 gehaltenen Verformungselements 12 vor der Mündung des Führungselements 26 steht. Dann wird das Vorschubelement aus seiner, das strumpfartige Netz 14 umschließenden vorgeschobenen Stellung in das Führungselement 26 zurückgezogen, so dass der zwischen den Endkappen 16 und 18 liegende Bereich des Netzes 14 aufweitbar wird. Diese Aufweitung in den in Figur 2 gezeigten aufgeweiteten Zustand erfolgt dann durch Zurückziehen des Zugorgans 22 nach außen, wodurch die äußere Endkappe 16 an die rückwärtige Endkappe 18 angenähert und das Netz 14 zwangsläufig im Sinne einer Verwölbung nach außen im Durchmesser vergrößert wird. Das sich aufweitende Netz verdrängt dabei das osteopathische Knochenmaterial und bildet einen Hohlraum, welcher - gegebenenfalls nach vorheriger Ausspülung von Knochenresten durch eine durch das schlauch- oder rohrförmige Führungselement eingebrachte und anschließend wieder abgesaugte Spül- und/oder Desinfektionsflüssigkeit - mit in noch bildsamem Zustand befindlichen abbindenden Knochenersatzmaterial gefüllt werden kann. Ein beim Absaugen der Spül-/Desinfektionsflüssigkeit im Hohlraum erzeugter leichter Unterdruck kann die Füllung des Hohlraums mit dem Knochenersatzmaterial fördern. Nach dem Abbinden oder Erhärten des Knochenersatzmaterials wird dann das Zugorgan 22 vom Instrument 10 abgekoppelt und zurückgezogen. Diese Abkopplung kann entweder durch Abtrennen des Zugorgans 22 entlang einer in der Nähe der vorderen Endkappe 16 vorgesehenen Sollbruchstelle durch Ausübung eines erhöhten Zugs erfolgen, oder das an seinem vorderen Ende mittels eines Gewindes in die Endkappe 16 eingeschraubten Zugorgan 22 wird herausgeschraubt und zurückgezogen.

Der durch das radial aufgeweitete Netz 14 gebildete Knochen-Hohlraum ist dann also nach Abbinden des Knochenersatzmaterials vollständig gefüllt, wodurch der Knochen insgesamt stabilisiert wird. Das im ausgehärteten Knochenmaterial verbleibende Netz 14 stellt eine zusätzliche Armierung für das abgebundene Knochenmaterial dar.

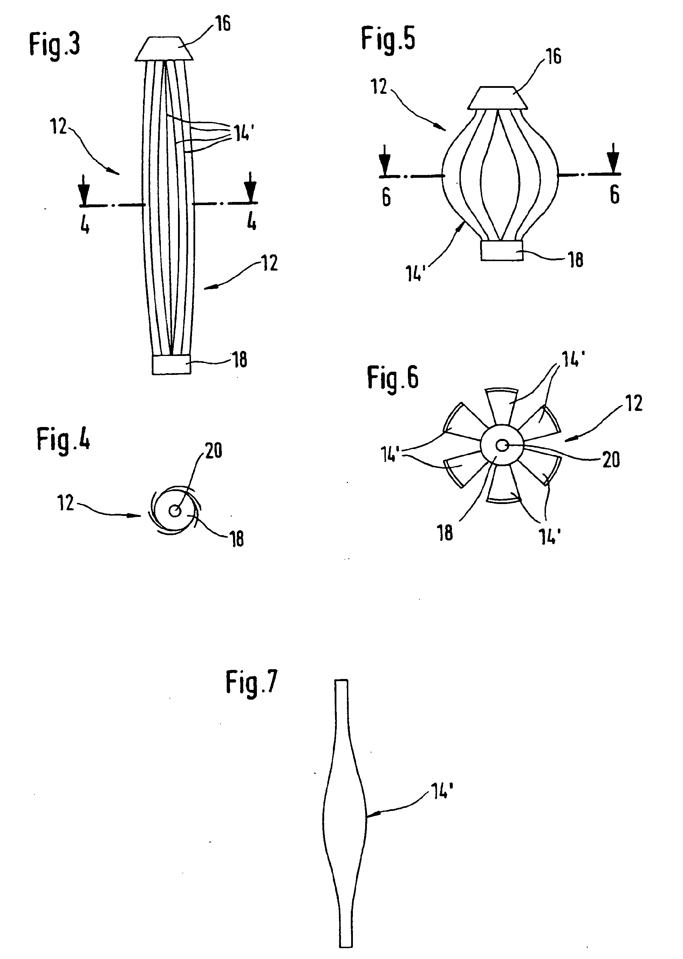

In den Figuren 3 bis 7 ist ein abgewandeltes Ausführungsbeispiel des Verformungselemens 12 dargestellt, welches sich von dem in Verbindung mit den Figuren 1 und 2 beschriebenen Verformungselements 12 im Wesentlichen dadurch unterscheidet, dass anstelle des strumpfartigen Netzes 14 aus Metalldrähten eine Vielzahl von jeweils mit ihren Enden in den Endkappen 16 und 18 befestigten metallischen Lamellen 14' vorgesehen sind, von denen eine Lamelle 14' in Figur 7 gesondert dargestellt ist. Diese Lamellen aus dünnem Metallblech, beispielsweise aus einem Titanblech, sind an ihren Enden jeweils in Umfangsrichtung zueinander versetzt in eine umlaufende Ringnut oder einzelne Einschnitte der Endkappen 16, 18 eingesetzt und dann in geeigneter Weise, z.B. durch Verlöten oder auch Verstemmen festgelegt. Die dünnen Lamellen 14' sind in ihren für die Befestigung vorgesehenen Endbereichen relativ schmal und verbreitern sich von ihren beiden Endbereichen in Richtung zum Mittelbereich, wodurch das Verformungselement 12 in der in den Figuren 5 und 6 ersichtlichen Weise in erheblich stärkerem Maße geschlossen ist, als das strumpfartige Netz 14 aus Metalldrähten des ersten Ausführungsbeispiels. Zwischen den Einzellamellen 14' bestehen in aufgeweitetern Zustand allerdings langgestreckte schlitzartige Durchlässe für das einzubringende Knochenersatzmaterial.

Die im Ausgangszustand sich im mittleren Bereich überdeckenden Lamellen 14' bilden im Bereich ihrer Randkanten im aufgeweiteten Zustand scharfe Kanten, die zur zusätzlichen Bearbeitung der Wandung des durch die Aufweitung gebildeten Hohlraums dienen können, indem das aufgeweitete Verformungselement zusätzlich im Knochen gedreht wird. Die Randkanten der Lamellen 14a schaben dann Knochengewebe von der Wandung des Hohlraums ab.

Es ist klar, dass bei dieser zusätzlichen Verwendung des Verformungskörpers 14 als materialabtragendes Werkzeug eine drehfeste Ankopplung der rückwärtigen Endkappe 18 am Vorschubelement 24 und/oder dem Führungselement 26 erfolgen muss. Dies kann beispielsweise durch entsprechende Vertiefungen im Randbereich der freien Stirnfläche der Endkappe 18 und Anordnung von komplementären Vorsprüngen am Vorschub- bzw. Führungselement erfolgen. Durch Drehen des Vorschub- bzw. Führungselements an seinem körperäußeren Ende kann dann - erforderlichenfalls - die angedeutete zusätzliche materialabtragende Bearbeitung des durch Aufweitung des Verformungselements 12 erzeugten Hohlraums erfolgen.

Darüber hinaus ist ersichtlich, dass im Rahmen des Erfindungsgedankens Abwandlungen und Weiterbildungen der vorstehend beschriebenen Ausführungsbeispiele verwirklichbar sind, die sich z.B. auf das für das Verformungselement verwendete Material beziehen. So können anstelle der erwähnten Materialabschnitte aus einer Titanlegierung auch anderen geeignete metallische Werkstoffe, z.B. Edelmetalle, Edelmetalllegierungen oder mit einer - beispielsweise galvanisch aufgebrachten - Edelmetallbeschichtung oder -plattierung versehene Materialabschnitte in Frage kommen. Auch aus hochreinem Glas, insbesondere Quarzglas, können die Materialabschnitte hergestellt sein, wobei dann durch eine geeignete Formgebung Sorge dafür getragen sein muss, dass die Materialabschnitte die für die radiale Aufweitung des Verstellelements erforderliche Verformbarkeit haben.

Auch aus thermoverformbaren Metallen, z.B. Bimetallelementen, denen elektrische Energie über am oder im Vorschubelement vorgesehene elektrische Leitungen zuführbar ist, hergestellte Materialabschnitte, können an die Stelle der beschriebenen Materialabschnitte aus Titan treten.

Um zusätzliche Informationen über das zu verdrängende und zu entfernende osteopathische Knochenmaterial und/oder den Fortschritt des Bearbeitungsprozesses mittels des erfindungsgemäßen Instruments zu erhalten, kann es weiter von Vorteil sein, wenn an oder in den Verstellelementen die Temperatur oder den Druck aufnehmende Sonden vorgesehen sind, deren Messsignale zu externen Mess- und/oder -verarbeitungseinrichtungen übertragen werden.

## Patentansprüche

1. Instrument (10) zur Behandlung von osteopathisch veränderten Bereichen der Wirbelsäule sowie von Röhrenknochen, welches ein am vorderen Ende eines schlauch- oder röhrchenartigen Führungselements (26) angeordnetes und mittels des Führungselements durch eine minimal-invasiv in der Kortikalis des Knochens erzeugte Öffnung ins Knocheninnere in den osteopathischen Knochenbereich einführ- und dort durch Manipulation von außen in seinen äußeren Abmessungen vergrößerbares Verformungselement (12) aufweist, welches bei Abmessungs-Vergrößerung durch Verdrängung von osteopathischem Knochenmaterial einen Hohlraum zu bilden vermag, in welchen aushärtendes oder abbindendes Knochen- und/oder Knochenersatzmaterial in noch bildsamen Zustand einbringbar ist, welches nach dem Abbinden oder Aushärten den osteopathischen Knochenbereich stabilisiert, wobei das Verformungselement (12) eine Vielzahl von in ihrem vorderen und rückwärtigen Endbereich gegen radiale Aufweitung miteinander verbundenen Abschnitte (14; 14') aus draht-, folien- oder dünnem plattenförmigen Material aufweist, welche in dem nicht in der äußeren Abmessung vergrößerten Zustand des Verformungselements einen radialen Außendurchmesser aufweisen, welcher ein Einführen durch den lichten Innendurchmesser des schlauch- oder röhrchenartigen Führungselements (26) erlaubt, und mit Mitteln zur Annäherung der miteinander verbundenen vorderen und rückwärtigen Endbereiche der Abschnitte (14; 14') des Verformungselements (12) derart versehen ist, dass sich diese zwischen ihren Endbereichen im Sinne einer radialen Vergrößerung des Durchmessers des Verformungselements (12) verformen, wobei das Verformungselement von lediglich in ihren gegenüberliegenden Endbereichen gegen radiale Aufweitung der miteinander verbundenen Abschnitten gebildet ist,
**dadurch gekennzeichnet,**
**dass** das
Verformungselement (12) in seiner im Ausgangszustand langgestreckten
Form in zurückgezogener Position in einem am vorderen Ende des schlauch- oder röhrchenartigen Führungselements (26) vorgesehenen schlauch- oder rohrförmigen Vorschubelement (24) und aus der zurückgezogen Position, in eine aus dem freien Ende des Vorschubelements (24) vorstehende Position verschiebbar angeordnet ist, und
**dass** das Vorschubelement (24) seinerseits aus einer in das Ende des Führungselements (26) zurückgezogenen in eine aus dem freien Ende des Führungselements (26) vorgeschobene Position verschiebbar im Führungselement (26) angeordnet ist.

2. Instrument nach Anspruch 1, **dadurch gekennzeichnet, dass** das Verformungselement von einem strumpfartigen Netz von Metalldrähten (14) gebildet wird, die im Bereich ihres vorderen und rückwärtigen Endes jeweils gegen radiale Aufweitung gesichert miteinander verbunden sind.

3. Instrument nach Anspruch 1, **dadurch gekennzeichnet, dass** das Verformungselement (12) von einer Anzahl von in Umfangsrichtung zueinander versetzten langgestreckten metallischen Folienabschnitten (14') gebildet wird, die in ihrem vorderen und rückwärtigen Endbereich gegen radiale Aufweitung gesichert miteinander verbunden sind.

4. Instrument nach einem der Ansprüche 1 bis 3, **dadurch gekennzeichnet, dass** die draht-, folien- oder plattenförmigen Materialabschnitte (14; 14') des Verformungselements (12) aus einem hochfesten Metall, vorzugsweise Titan oder einer Titanlegierung hergestellt sind.

5. Instrument nach Anspruch 1, **dadurch gekennzeichnet, dass** die Materialabschnitte des Verformungselements (12) aus hochgereinigtem Glas, vorzugsweise Quarzglas, hergestellt sind.

6. Instrument nach einem der Ansprüche 1 bis 3, **dadurch gekennzeichnet, dass** die Materialabschnitte (14; 14') des Verformungselements (12) aus thermoverformbarem Metall, vorzugsweise einem Edelmetall, hergestellt sind.

7. Instrument nach Anspruch 6, **dadurch gekennzeichnet, dass** die Materialabschnitte (14; 14') des Verformungselements (12) als Bimetallelement ausgebildet ist.

8. Instrument nach Anspruch 6, **dadurch gekennzeichnet, dass** die Materialabschnitte (14; 14') des Verformungselements (12) aus einem Metall hergestellt sind, welches Memory-Effekt-Materialeigenschaften aufweist.

9. Instrument nach einem der Ansprüche 6 bis 8, **dadurch gekennzeichnet, dass** am oder im Verformungselement (12) eine an eine äußere Temperaturmess- und/oder -verarbeitungseinrichtung angeschlossene Temperatursonde vorgesehen ist.

10. Instrument nach einem der Ansprüche 6 bis 9, **dadurch gekennzeichnet, dass** am oder im Verformungselement (12) eine an eine äußere Druckmess- und/oder -verarbeitungseinrichtung angeschlossene Drucksonde vorgesehen ist.

11. Instrument nach Anspruch 10, **dadurch gekennzeichnet, dass** die Drucksonde einen piezoelektrischen Druckaufnehmer aufweist.

12. Instrument nach einem der Ansprüche 1 bis 11, **dadurch gekennzeichnet, dass** das Verformungselement (12) von einem an dessen vorderen, gegen radiale Aufweitung gesicherten Endbereich befestigten und durch den rückwärtigen Endbereich verschieblich bis aus dem äußeren Ende des schlauch- oder röhrchenförmigen Führungselements herausgeführtes Zugorgan (22) durchsetzt wird.

13. Instrument nach Anspruch 12, **dadurch gekennzeichnet, dass** das Zugorgan (22) in seinem im Ausgangszustand innerhalb des Verformungselements (12) verlaufenden Bereich mit einer Sollbruchstelle versehen ist.

14. Instrument nach Anspruch 12, **dadurch gekennzeichnet, dass** das Zugorgan (22) mittels einer lösbaren Kupplung am vorderen Endbereich des Verformungselements (12) befestigt ist.

15. Instrument nach einem der Ansprüche 1 bis 14, **dadurch gekennzeichnet, dass** die das Verformungselement (12) bildenden Materialabschnitte (14; 14') aus biegeelastischem Material hergestellt sind und in unverformtem Zustand eine der radial aufgeweiteten Form des Verformungselements entsprechende Form haben.

## Claims

1. Instrument (10) for the treatment of osteopathically altered regions of the spinal column and also of long bones, comprising a deformable element (12) which is disposed on the anterior end of a hose-like or tubular guide element (26) and is introduced by means of the guide element through a minimally invasively produced opening in the cortical bone into the interior of the bone in the osteopathic region of the bone, where its external dimensions can be enlarged by manipulation from the exterior, wherein when the dimensions of the deformable element (12) are enlarged the instrument can by displacement of osteopathic bone material form a cavity into which hardening or setting bone and/or bone substitute material can be introduced in the state in which it is still workable and after setting or hardening stabilises the osteopathic bone region, wherein the deformable element (12) has a plurality of sections (14; 14') made from material in the form of wires, films or thin plates which are joined to one another in the anterior and posterior region thereof to counter radial expansion, wherein in the state of the deformable element in which the external dimensions are not enlarged this plurality of sections have a radial external diameter which allows introduction through the clear internal diameter of the hose-like or tubular guide element (26), and is provided with means for approximation of the anterior and posterior end regions of the sections (14; 14') of the deformable element (12) which are joined together, in such a way that these sections deform between their end regions to produce a radial enlargement of the diameter of the deformable element (12), wherein the deformable element is formed by sections which are joined to one another only in their opposing end regions to counter radial expansion, **characterised in that** the deformable element (12) which in its initial state is extended is disposed in the retracted position in a hose-like or tubular advancing element (24) provided on the anterior end of the hose-like or tubular guide element (26) and is displaceable out of the retracted position into a position in which it projects out of the free end of the advancing element (24), and **in that** the advancing element (24) for its part is disposed so that it is displaceable in the guide element (26) out of a position in which it is retracted into the end of the guide element (26) and a position in which it is advanced out of the free end of the guide element (26).

2. Instrument as claimed in Claim 1, **characterised in that** the deformable element is formed by a hose-like mesh of metal wires (14) which are joined securely to one another in the region of its anterior and posterior end in each case to counter radial expansion.

3. Instrument as claimed in Claim 1, **characterised in that** the deformable element (12) is formed by a plurality of elongate metallic film sections (14') which are offset with respect to one another in the peripheral direction and are securely joined to one another in their anterior and posterior end regions to counter radial expansion.

4. Instrument as claimed in any one of Claims 1 to 3, **characterised in that** the material sections (14; 14') of the deformable element (12) in the form of wires, films or plates are produced from a high-strength metal, preferably titanium or a titanium alloy.

5. Instrument as claimed in Claim 1, **characterised in that** the material sections of the deformable element (12) are produced from highly purified glass, preferably vitreous silica.

6. Instrument as claimed in any one of Claims 1 to 3, **characterised in that** the material sections (14; 14') of the deformable element (12) are produced from thermodeformable metal, preferably a precious metal.

7. Instrument as claimed in Claim 6, **characterised in that** the material sections (14; 14') of the deformable element (12) are constructed as bimetallic elements.

8. Instrument as claimed in Claim 6, **characterised in that** the material sections (14; 14') of the deformable element (12) are produced from a metal which has memory effect material characteristics.

9. Instrument as claimed in any one of Claims 6 to 8, **characterised in that** a temperature probe connected to an external temperature measuring and/or processing device is provided on or in the deformable element (12).

10. Instrument as claimed in any one of Claims 6 to 9, **characterised in that** a pressure probe connected to an external pressure measuring and/or processing device is provided on or in the deformable element (12).

11. Instrument as claimed in Claim 10, **characterised in that** the pressure probe is a piezoelectric pressure sensor.

12. Instrument as claimed in any one of Claims 1 to 11, **characterised in that** a traction member (22), which is fixed on the anterior end region of the deformable element (12) secured against radial expansion and which is displaceable through the posterior end region until it extends out of the outer end of the hose-like or tubular guide element, extends through the deformable element (12).

13. Instrument as claimed in Claim 12, **characterised in that** the traction member (22) is provided with a predetermined breaking point in its region which in the initial state extends inside the deformable element (12).

14. Instrument as claimed in Claim 12, **characterised in that** the traction member (22) is fixed on the anterior end region of the deformable element (12) by means of a releasable coupling.

15. Instrument as claimed in any one of Claims 1 to 14, **characterised in that** the material sections (14; 14') forming the deformable element (12) are produced from resilient material and in the undeformed state have a shape corresponding to the radially expanded shape of the deformable element.

## Revendications

1. Instrument (10) de traitement de zones modifiées au plan ostéopathique de la colonne vertébrale et d'os longs, qui présente un élément de déformation (12) agencé à l'extrémité avant d'un élément de guidage (26) en forme de tuyau ou de tube et pouvant être introduit au moyen de l'élément de guidage à travers une ouverture générée par effraction minimale dans la corticale de l'os à l'intérieur de l'os dans la zone osseuse ostéopathique et pouvant y être agrandi dans ses dimensions externes par manipulation de l'extérieur, élément qui peut former une cavité lors de l'agrandissement des dimensions par déplacement du matériau osseux ostéopathique, dans laquelle cavité il peut être introduit un matériau osseux et/ou un matériau de substitution osseux à l'état encore malléable pouvant durcir ou prendre, qui stabilise la zone osseuse ostéopathique après la prise ou le durcissement, dans lequel l'élément de déformation (12) présente une pluralité de sections (14 ; 14') reliées l'une à l'autre dans leurs zones d'extrémité avant et arrière à l'encontre d'un élargissement radial et constituées d'un matériau en forme de fil, de feuille ou de plaque mince, qui présentent, à l'état non agrandi des dimensions externes de l'élément de déformation, un diamètre radial externe qui permet une introduction à travers le diamètre intérieur de l'élément de guidage (26) en forme de tuyau ou de tube, et est pourvu de moyens de rapprochement des zones d'extrémité avant et arrière, reliées l'une à l'autre, des sections (14 ; 14') de l'élément de déformation (12) de sorte que celles-ci se déforment entre leurs zones d'extrémité dans le sens d'un agrandissement radial du diamètre de l'élément de déformation (12), l'élément de déformation étant formé uniquement dans ses zones d'extrémité opposées contre un élargissement radial des sections reliées l'une à l'autre,
**caractérisé en ce que**
l'élément de déformation (12) est agencé à coulissement
dans sa forme allongée à l'état initial en position rétractée dans un élément d'avancement (24) en forme de tuyau ou de tube prévu à l'extrémité avant de l'élément de guidage (26) en forme de tuyau ou de tube et, à partir de la position rétractée, dans une position faisant saillie de l'extrémité libre de l'élément d'avancement (24), et
**en ce que** l'élément d'avancement (24) est agencé à son tour
à coulissement dans l'élément de guidage (26) d'une position rétractée dans l'extrémité de l'élément de guidage (26) à une position avancée depuis l'extrémité libre de l'élément de guidage (26).

2. Instrument selon la revendication 1, **caractérisé en ce que** l'élément de déformation est formé d'un réseau en forme de bas de fils métalliques (14), qui sont solidement reliés l'un à l'autre respectivement contre un élargissement radial dans la zone de leurs extrémités avant et arrière.

3. Instrument selon la revendication 1, **caractérisé en ce que** l'élément de déformation (12) est formé d'un certain nombre de sections de feuille métalliques (14') allongées décalées l'une de l'autre dans la direction périphérique, qui sont solidement reliées l'une à l'autre dans leur zone d'extrémité avant et arrière contre un élargissement radial.

4. Instrument selon l'une quelconque des revendications 1 à 3, **caractérisé en ce que** les sections de matériau (14 ; 14') en forme de fil, de feuille ou de plaque mince de l'élément de déformation (12) sont fabriquées en métal hautement résistant, de préférence du titane ou un alliage de titane.

5. Instrument selon la revendication 1, **caractérisé en ce que** les sections de matériau de l'élément de déformation (12) sont fabriquées en verre hautement purifié, de préférence du verre de quartz.

6. Instrument selon l'une quelconque des revendications 1 à 3, **caractérisé en ce que** les sections de matériau (14 ; 14') de l'élément de déformation (12) sont fabriquées en métal thermoformable, de préférence un métal noble.

7. Instrument selon la revendication 6, **caractérisé en ce que** les sections de matériau (14 ; 14') de l'élément de déformation (12) se présentent sous la forme d'un élément bimétallique.

8. Instrument selon la revendication 6, **caractérisé en ce que** les sections de matériau (14 ; 14') de l'élément de déformation (12) sont fabriquées en métal à propriétés de matériau à effet de mémoire.

9. Instrument selon l'une quelconque des revendications 6 à 8, **caractérisé en ce qu'**il est prévu, sur ou dans l'élément de déformation (12), une sonde de température raccordée à un dispositif de mesure et/ou de traitement de température externe.

10. Instrument selon l'une quelconque des revendications 6 à 9, **caractérisé en ce qu'**il est prévu, sur ou dans l'élément de déformation (12), une sonde de pression raccordée à un dispositif de mesure et/ou de traitement de pression externe.

11. Instrument selon la revendication 10, **caractérisé en ce que** la sonde de pression présente un capteur de pression piézoélectrique.

12. Instrument selon l'une quelconque des revendications 1 à 11, **caractérisé en ce que** l'élément de déformation (12) est traversé par un organe de traction (22) fixé sur sa zone d'extrémité protégée contre un élargissement radial et dégagé à travers la zone d'extrémité à coulissement jusqu'à l'extrémité externe de l'élément de guidage en forme de tuyau ou de tube.

13. Instrument selon la revendication 12, **caractérisé en ce que** l'organe de traction (22) est pourvu d'un point de rupture théorique dans sa zone s'étendant à l'état initial à l'intérieur de l'élément de déformation (12).

14. Instrument selon la revendication 12, **caractérisé en ce que** l'organe de traction (22) est fixé au moyen d'un couplage détachable sur la zone d'extrémité avant de l'élément de déformation (12).

15. Instrument selon l'une quelconque des revendications 1 à 14, **caractérisé en ce que** les sections de matériau (14 ; 14') formant l'élément de déformation sont fabriquées en matériau élastique à la flexion et ont, à l'état non déformé, une forme correspondant à la forme radialement élargie de l'élément de déformation.
